(19) Europäisches Patentamt
European Patent Office
Office européen des brevets

(11) Veröffentlichungsnummer: **0 040 740**
**B1**

# (12) EUROPÄISCHE PATENTSCHRIFT

(45) Veröffentlichungstag der Patentschrift: **14.11.84**

(21) Anmeldenummer: **81103579.9**

(22) Anmeldetag: **11.05.81**

(51) Int. Cl.³: **C 07 C 87/28**, C 07 C 91/16, C 07 D 265/30, C 07 D 295/08, C 07 F 7/18, A 01 N 33/04, A 01 N 33/08, A 01 N 43/34, A 01 N 43/84, A 01 N 55/00

## (54) Aminopropanol-Derivate, Verfahren zu ihrer Herstellung und ihre Verwendung als Fungizide.

(30) Priorität: **22.05.80 DE 3019496**

(43) Veröffentlichungstag der Anmeldung: **02.12.81 Patentblatt 81/48**

(45) Bekanntmachung des Hinweises auf die Patenterteilung: **14.11.84 Patentblatt 84/46**

(84) Benannte Vertragsstaaten: **AT BE CH DE FR GB IT LI NL SE**

(56) Entgegenhaltungen: ***keine***

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Knops, Hans-Joachim, Dr., Claudiusweg 3, D-5600 Wuppertal-1 (DE)**
Erfinder: **Krämer, Wolfgang, Dr., Am Eckbusch 39/45, D-5600 Wuppertal-1 (DE)**
Erfinder: **Frohberger, Paul-Ernst, Dr., Willi-Baumeister-Strasse 5, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue Aminopropanol-Derivate, mehrere Verfahren zu ihrer Herstellung und ihre Verwendung als Fungizide.

Es ist bereits bekannt geworden, dass Triazolyl-ethanol-Derivate, wie z.B. 1-(4-Chlorphenyl)-2-(1,2,4-triazol-1-yl)-1-ethanol, im allgemeinen gute fungizide Eigenschaften aufweisen (vgl. DE-OS 2 431 407 [Le A 15 735]). Die Wirkung ist jedoch, insbesondere bei niedrigen Aufwandmengen und -konzentrationen in einigen Anwendungsbereichen nicht immer voll befriedigend.

Weiterhin sind Piperidin-, Morpholin- und Piperidon-Derivate, wie z.B. das 1-[3-(p-tert.-Butylphenyl)-3-hydroxy-2-methyl-propyl]-piperidin und deren Verwendung als Fungizide bekannt (vgl. EP-A 5541).

Es wurden neue Aminopropanol-Derivate der allgemeinen Formel

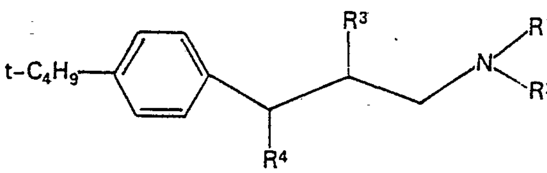

(I)

in welcher

$R^1$ und $R^2$ für Alkyl mit 1 bis 4 Kohlenstoffatomen stehen; oder gemeinsam mit dem Stickstoffatom, an das sie gebunden sind, für die gegebenenfalls durch Alkyl mit 1 bis 4 Kohlenstoffatomen sowie durch einen gegebenenfalls durch Alkyl mit 1 bis 4 Kohlenstoffatomen und Halogen substituierten ankondensierten aromatischen oder alicyclischen Ring mit 5 bis 7 Kohlenstoffatomen substituierten Heterocyclen

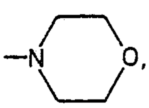, 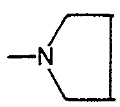 und 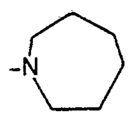

stehen; sowie.

zusätzlich auch für den gegebenenfalls durch Alkyl mit 1 bis 4 Kohlenstoffatomen sowie durch einen gegebenenfalls durch Alkyl mit 1 bis 4 Kohlenstoffatomen und Halogen substituierten ankondensierten aromatischen oder alicyclischen Ring mit 5 bis 7 Kohlenstoffatomen substituierten Heterocyclus

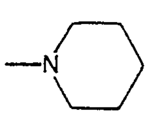

stehen, wenn $R^4$ für die Reste $-O-CO-R^5$, $-O-CO-NHR^6$ oder $-O-SiR_3{}^7$ steht,

$R^3$ für Wasserstoff oder Alkyl mit 1 bis 4 Kohlenstoffatomen steht,

$R^4$ für die Hydroxygruppe, Halogen sowie die Reste $-O-CO-R^5$, $-O-CO-NHR^6$ oder $-O-SiR_3{}^7$ steht,

$R^5$ für Alkyl mit 1 bis 4 Kohlenstoffatomen, Alkenyl mit 2 bis 4 Kohlenstoffatomen, Halogenalkyl mit 1 bis 2 Kohlenstoffatomen und 1 bis 5 gleichen oder verschiedenen Halogenatomen, sowie für gegebenenfalls substituiertes Aryl mit 6 bis 10 Kohlenstoffatomen und Aralkyl mit 6 bis 10 Kohlenstoffatomen im Arylteil und 1 bis 2 Kohlenstoffatomen im Alkylteil, wobei als Substituenten genannt seien: Halogen, Alkyl mit 1 bis 4 Kohlenstoffatomen, Halogenalkyl mit 1 bis 2 Kohlenstoffatomen und 1 bis 5 gleichen oder verschiedenen Halogenatomen, Alkoxy und Alkylthio mit jeweils 1 oder 2 Kohlenstoffatomen steht,

$R^6$ für Alkyl mit 1 bis 8 Kohlenstoffatomen, Alkenyl mit 2 bis 4 Kohlenstoffatomen sowie für gegebenenfalls substituiertes Aryl mit 6 bis 10 Kohlenstoffatomen, wobei als Substituenten die bereits obengenannten Phenylsubstituenten infrage kommen und

$R^7$ für Alkyl mit 1 bis 4 Kohlenstoffatomen steht,

und deren physiologisch verträglichen Säureadditions-Salze gefunden.

Weiterhin wurde gefunden, dass man die Aminopropanol-Derivate der Formel (I) erhält, wenn man

a) Aminopropiophenone der Formel

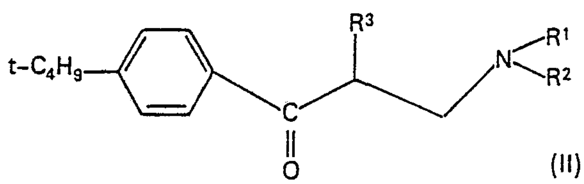

(II)

in welcher

$R^1$, $R^2$ und $R^3$ die oben angegebene Bedeutung haben,

mit komplexen Hydriden in Gegenwart eines Lösungsmittels reduziert, und

gegebenenfalls die nach dem Verfahren a) erhältlichen Aminopropanole der Formel

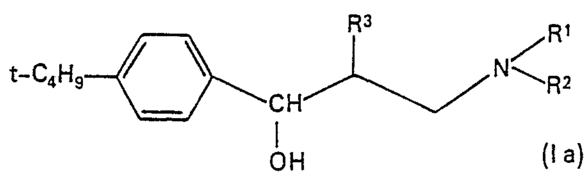

(I a)

b) mit einem Halogenierungsmittel, gegebenenfalls in Gegenwart eines Lösungsmittels, und gegebenenfalls in Gegenwart eines Säurebindemittels, umsetzt, oder

c) mit Halogeniden der Formel

$$Hal-R^8 \qquad (III)$$

in welcher

$R^8$ für die Reste $-CO-R^5$, $-CO-NHR^6$ oder $-SiR_3{}^7$ steht, wobei $R^5$, $R^6$ und $R^7$ die oben angegebene Bedeutung haben und

Hal für Halogen steht,

in Gegenwart eines Lösungsmittels und gegebenenfalls in Gegenwart einer starken Base bzw. gegebenenfalls in Gegenwart eines Säurebindemittels umsetzt, oder

d) mit Säureanhydriden der Formel

$R^5$-CO-O-CO-$R^5$ (IV)

in welcher

$R^5$ die oben angegebene Bedeutung hat,

in Gegenwart eines Lösungsmittels und gegebenenfalls in Gegenwart eines Katalysators umsetzt, oder

e) mit Isocyanaten der Formel

O=C=N-$R^6$ (V)

in welcher

$R^6$ die oben angegebene Bedeutung hat,

in Gegenwart eines Lösungsmittels und gegebenenfalls in Gegenwart eines Katalysators umsetzt,

und gegebenenfalls an die nach obigen Verfahren erhaltenen Verbindungen der Formel (I) eine physiologisch verträgliche Säure addiert.

Die neuen Aminopropanol-Derivate der Formel (I) weisen starke fungizide Eigenschaften auf. Dabei zeigen überraschenderweise die erfindungsgemässen Verbindungen eine höhere Wirkung als das aus dem Stand der Technik bekannte 1-(4-Chlorphenyl)-2-(1,2,4-triazol-1-yl)-1-ethanol; welches eine Verbindung gleicher Wirkungsrichtung ist. Die erfindungsgemässen Stoffe stellen somit eine Bereicherung der Technik dar.

Die erfindungsgemässen Aminopropanol-Derivate sind durch die Formel (I) allgemein definiert. In dieser Formel sind $R^1$ und $R^2$ gleich oder verschieden und stehen vorzugsweise für geradkettiges oder verzweigtes Alkyl mit 1 bis 4 Kohlenstoffatomen. $R^1$ und $R^2$ stehen ausserdem gemeinsam mit dem Stickstoffatom, an das sie gebunden sind, vorzugsweise für die gegebenenfalls substituierten Heterocyclen

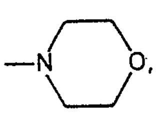, 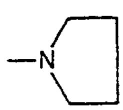 und 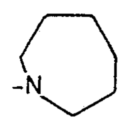

sowie zusätzlich auch für den gegebenenfalls substituierten Heterocyclus

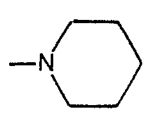 ,

wenn $R^4$ für die Reste -O-CO-$R^5$, -O-CO-NH$R^6$ oder -O-Si$R_3^7$ steht, wobei als Substituenten vorzugsweise infrage kommen: Alkyl mit 1 bis 4 Kohlenstoffatomen sowie ein gegebenenfalls durch Alkyl mit 1 bis 4 Kohlenstoffatomen und Halogen substituierter ankondensierter aromatischer oder alicyclischer Ring mit 5 bis 7 Kohlenstoffatomen.

$R^3$ steht vorzugsweise für Wasserstoff oder Alkyl mit 1 bis 4 Kohlenstoffatomen.

$R^4$ steht vorzugsweise für die Hydroxygruppe, für Chlor und Brom, sowie für die Reste -O-CO-$R^5$, -O-CO-NH$R^6$ und -O-Si$R_3^7$.

$R^5$ steht vorzugsweise für geradkettiges oder verzweigtes Alkyl mit 1 bis 4 Kohlenstoffatomen, Alkenyl mit 2 bis 4 Kohlenstoffatomen, Halogenalkyl mit 1 bis 2 Kohlenstoffatomen und 1 bis 5 gleichen oder verschiedenen Fluor- und Chloratomen, sowie für gegebenenfalls substituiertes Aryl mit 6 bis 10 Kohlenstoffatomen und Aralkyl mit 6 bis 10 Kohlenstoffatomen im Arylteil und 1 bis 2 Kohlenstoffatomen im Alkylteil, wie insbesondere Phenyl und Benzyl, wobei als Substituenten vorzugsweise infrage kommen: Halogen, Alkyl mit 1 bis 4 Kohlenstoffatomen, Halogenalkyl mit 1 bis 2 Kohlenstoffatomen und 1 bis 5 gleichen oder verschiedenen Fluor- und Chloratomen, sowie Alkoxy und Alkylthio mit jeweils 1 oder 2 Kohlenstoffatomen.

$R^6$ steht vorzugsweise für geradkettiges oder verzweigtes Alkyl mit 1 bis 8 Kohlenstoffatomen, Alkenyl mit 2 bis 4 Kohlenstoffatomen sowie für gegebenenfalls substituiertes Aryl mit 6 bis 10 Kohlenstoffatomen, wie insbesondere Phenyl, wobei als Substituenten vorzugsweise die bereits obengenannten Phenylsubstituenten infrage kommen und

$R^7$ steht vorzugsweise für geradkettiges oder verzweigtes Alkyl mit 1 bis 4 Kohlenstoffatomen.

Besonders bevorzugt sind diejenigen Aminopropanol-Derivate der Formel (I), in denen $R^1$ und $R^2$ für Alkyl mit 1 bis 4 Kohlenstoffatomen stehen, gemeinsam mit dem Stickstoffatom, an das sie gebunden sind, für die gegebenenfalls substituierten Heterocyclen

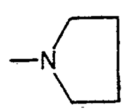 und 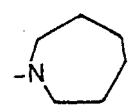

stehen, sowie zusätzlich auch für die gegebenenfalls substituierten Heterocyclen

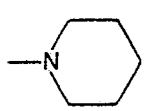, und 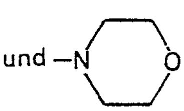 stehen,

wenn $R^4$ für die Reste -O-CO-$R^5$, -O-CO-NH$R^6$ oder -O-Si$R_3^7$ steht, wobei als Substituenten infrage kommen: Methyl, Ethyl, sowie ein ankondensierter Benzol- oder Cyclohexylring; $R^3$ für Wasserstoff oder Methyl steht; $R^4$ für die Hydroxygruppe, Chlor, Brom sowie die Reste -O-CO-$R^5$, -O-CO-NH$R^6$ und -OSi$R_3^7$ steht; $R^5$ für Methyl, Ethyl, Osopropyl, Isobutyl, Chlormethyl, Dichlormethyl, oder gegebenenfalls einfach oder mehrfach substituiertes Phenyl und Benzyl mit Chlor, Brom, Methyl oder Trifluormethyl als Substituenten steht; $R^6$ für geradkettiges oder verzweigtes Alkyl mit 1 bis 4 Kohlenstoffatomen oder gegebenenfalls einfach oder mehrfach substituiertes Phenyl mit Chlor, Brom, Methyl oder Trifluormethyl als Substituenten steht und $R^7$ für Methyl oder Ethyl steht.

Im einzelnen seien ausser den beiden Herstellungsbeispielen genannten Verbindungen die folgenden Verbindungen der allgemeinen Formel (I) genannt:

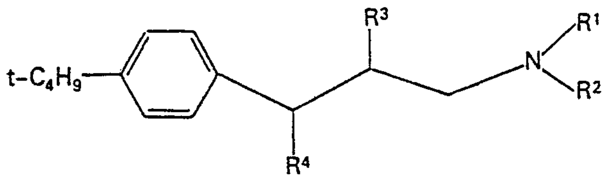
(I)

| $R^4$ | $R^3$ | 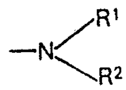 |
|---|---|---|
| $-O-CO-CH_2Cl$ | $CH_3$ | 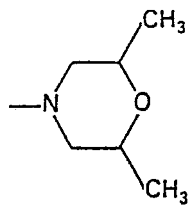 |
| $-O-CO-CH\ Cl_2$ | $CH_3$ | 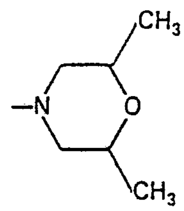 |
| -O-CO-NH- 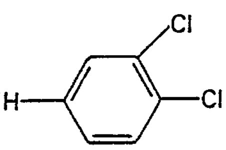 | $CH_3$ | 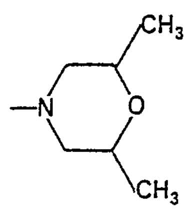 |
| Cl | $CH_3$ | 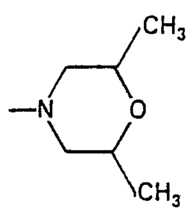 |
| -O-CO-NH- 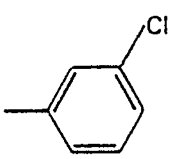 | $CH_3$ | 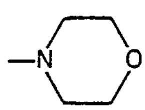 |
| -O-CO-NH- 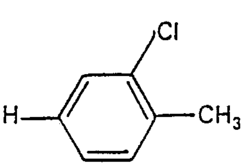 | $CH_3$ | 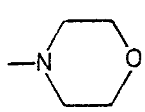 |

| $R^4$ | $R^3$ | 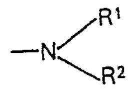 |
|---|---|---|
| -O-CO-NH- 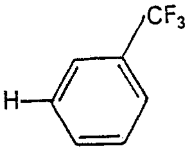 | $CH_3$ | 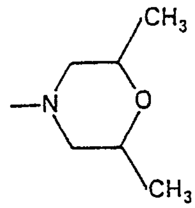 |
| $-O-CO-NH-C_3H_7$-iso | $CH_3$ | 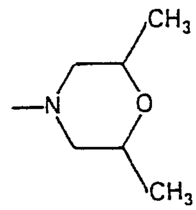 |
| $-O-CO-NH-CH_3$ | $CH_3$ | 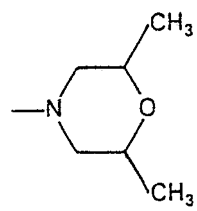 |
| $-O-CO-NH-C_4H_9$-iso | $CH_3$ | 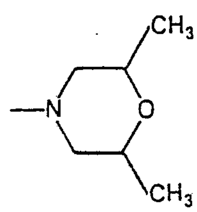 |
| $-O-CO-NH-C_4H_9$-n | $CH_3$ | 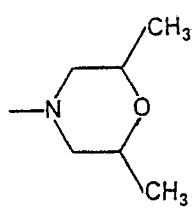 |
| $-O-CO-NH-C_4H_9$-n | $CH_3$ | 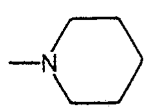 |
| $-O-CO-NH-C_4H_9$-iso | $CH_3$ | 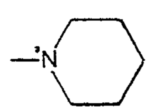 |
| $-O-CO-NH-CH_3$ | $CH_3$ | 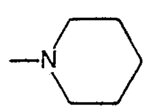 |

| R4 | R3 | 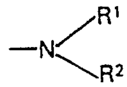 |
|---|---|---|
| $-O-CO-NH-C_3H_7$-iso | $CH_3$ | 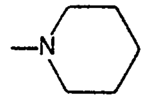 |
| -O-CO-NH 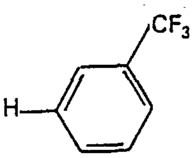 | $CH_3$ | 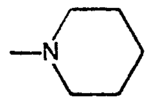 |
| -O-CO-NH 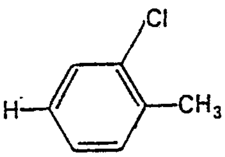 | $CH_3$ | 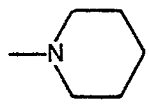 |
| -O-CO-NH 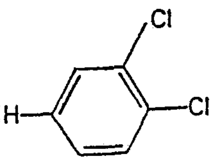 | $CH_3$ | 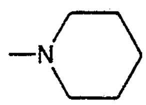 |
| -OH | $CH_3$ | 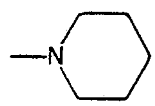 |
| $-O-CO-CHCl_2$ | $CH_3$ | 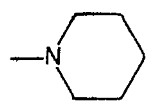 |
| $-O-CO-CH_2Cl$ | $CH_3$ | 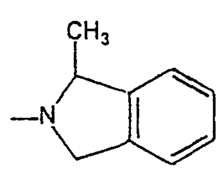 |
| -OH | $CH_3$ | $-N(C_2H_5)_2$ |
| -OH | $CH_3$ | $-N(C_3H_7$-iso$)_2$ |
| -OH | $CH_3$ | $-N(C_3H_7$-n$)_2$ |
| -OH | $CH_3$ | $-N(C_4H_9$-n$)_2$ |
| -OH | $CH_3$ | $-N(C_4H_9$-iso$)_2$ |

Verwendet man p-tert.-Butyl-2-methyl-3-morpholin-4-yl-propiophenon und Natriumborhydrid als Ausgangsstoffe, so kann der Reaktionsablauf durch das folgende Formelschema wiedergegeben werden (Verfahren a):

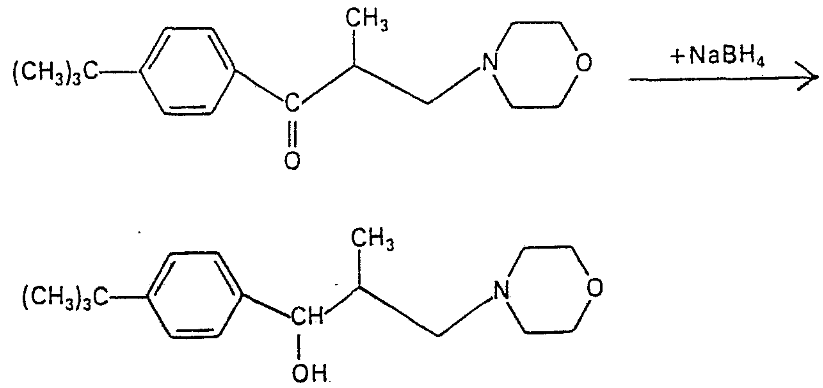


Verwendet man 1-(p-tert.-Butyl-phenyl)-2-methyl-3-morpholin-4-yl-1-propanol und Thionylchlorid als Ausgangsstoffe, so kann der Reaktionsablauf durch das folgende Formelschema wiedergegeben werden (Verfahren b):

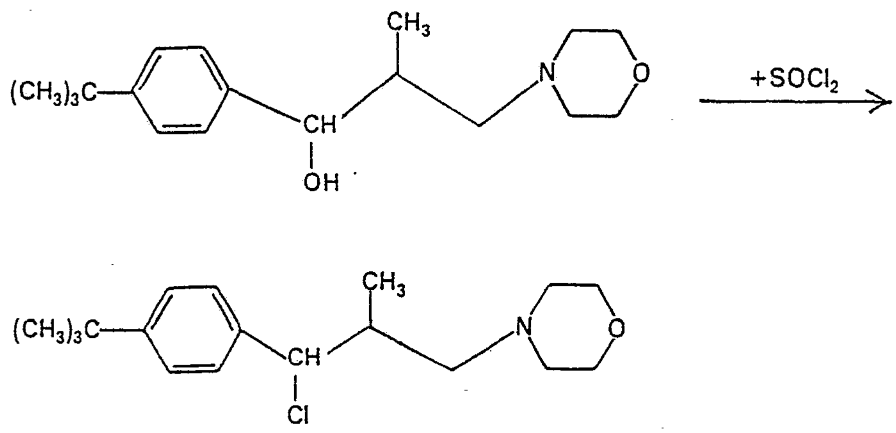


Verwendet man 1-(p-tert.-Butyl-phenyl)-2-methyl-3-morpholin-4-yl-1-propanol und Acetylchlorid als Ausgangsstoffe, so kann der Reaktionsablauf durch das folgende Formelschema wiedergegeben werden (Verfahren c):

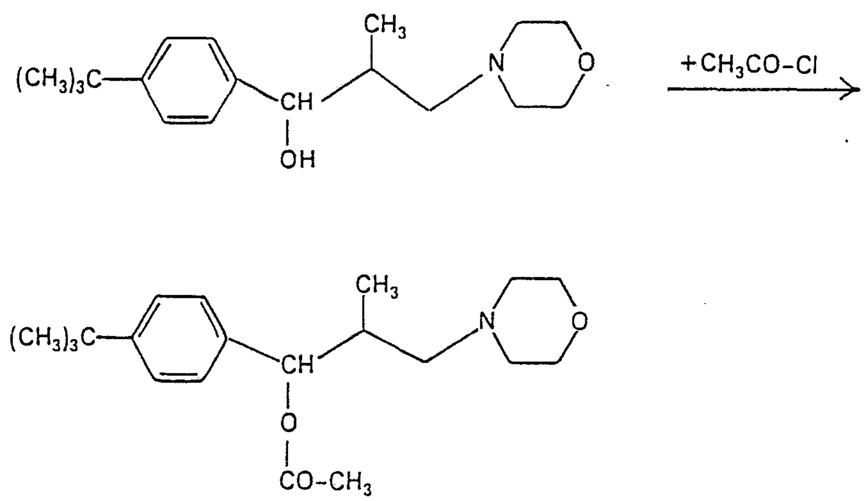


Verwendet man 1-(p-tert.-Butyl-phenyl)-2-methyl-3-morpholin-4-yl-1-propanol und Trimethylchlorsilan als Ausgangsstoffe, so kann der Reaktionsablauf durch das folgende Formelschema wiedergegeben werden (Verfahren c):

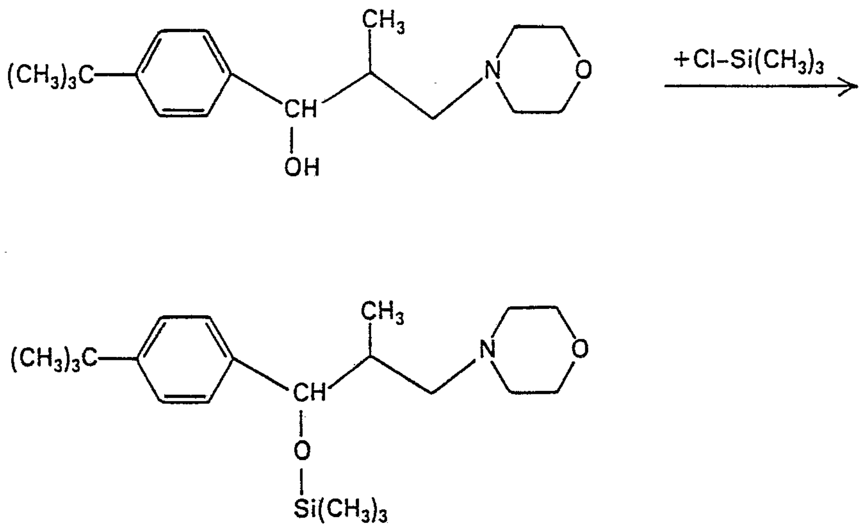


Verwendet man 1-(p-tert.-Butyl-phenyl)-2-methyl-3-morpholin-4-yl-1-propanol und Essigsäureanhydrid als Ausgangsstoffe, so kann der Reaktionsablauf durch das folgende Formelschema wiedergegeben werden (Verfahren d):

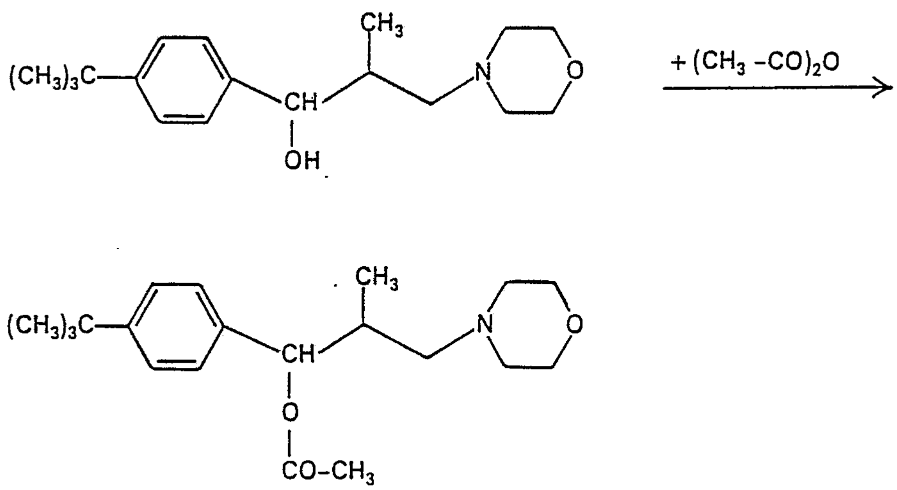


Verwendet man 1-(p-tert.-Butyl-phenyl)-2-methyl-3-morpholin-4-yl-1-propanol und Phenylisocyanat als Ausgangsstoffe, so kann der Reaktionsablauf durch das folgende Formelschema wiedergegeben werden (Verfahren e):

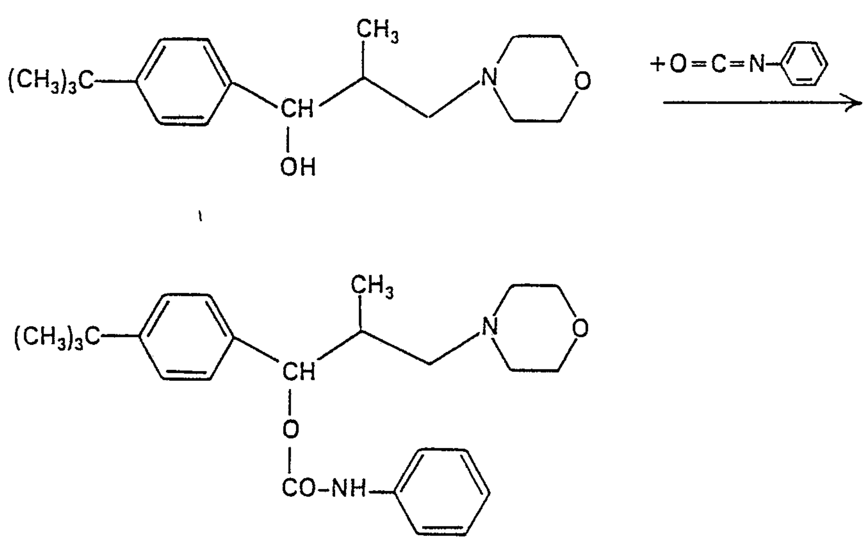

Die für die erfindungsgemässe Verfahren (a) als Ausgangsstoffe zu verwendenden Aminopropiophenone sind durch die Formel (II) allgemein definiert. In dieser Formel stehen $R^1$, $R^2$ und $R^3$ vorzugsweise für diejenigen Reste, die bereits im Zusammenhang mit der Beschreibung der erfindungsgemässen Stoffe der Formel (I) vorzugsweise für diese Substituenten genannt wurden.

Die Aminopropiophenone der Formel II sind teilweise bekannt (vgl. veröffentlichte Europäische Patentanmeldung Nr. 0 005 541); sie werden erhalten, indem man bekannte Acetophenone der Formel

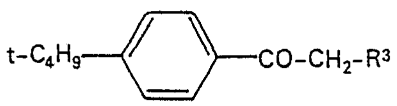

(VI)

in welcher
$R^3$ die oben angegebene Bedeutung hat,
mit Paraformaldehyd und mit Aminen der Formel

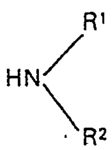

(VII)

in welcher
$R^1$ und $R^2$ die oben angegebene Bedeutung haben, in Gegenwart eines protischen Lösungsmittels, wie beispielsweise Ethanol, bei Temperaturen zwischen 50 und 120°C umsetzt, wobei die Amine der Formel (VII) vorzugsweise als Hydrochloride eingesetzt werden.

Die ausserdem für das erfindungsgemässe Verfahren (a) als Ausgangsstoffe benötigten komplexen Hydride sind allgemein bekannte Verbindungen der organischen Chemie. Als Beispiele seien vorzugsweise Natriumborhydrid und Lithiumalanat genannt.

Die für die erfindungsgemässen Verfahren (b), (c), (d) und (e) als Ausgangsstoffe zu verwendenden Aminopropanole der Formel (Ia) sind erfindungsgemässe Verbindungen.

Die ausserdem für das erfindungsgemässe Verfahren (b) benötigten Halogenierungsmittel sind allgemein bekannte Verbindungen der organischen Chemie. Als Beispiele seien vorzugsweise anorganische Säurehalogenide genannt, wie Phosphortrichlorid, -tribromid und -pentachlorid, Phosphoroxychlorid und Thionylchlorid.

Die ausserdem für das erfindungsgemässe Verfahren (c) als Ausgangsstoffe zu verwendenden Halogenide sind durch die Formel (III) allgemein definiert. In dieser Formel steht $R^8$ vorzugsweise für die Reste $-CO-R^5$, $-CO-NHR^6$ und $-SiR_3{}^7$, wobei $R^5$, $R^6$ und $R^7$ vorzugsweise für diejenigen Reste stehen, die bereits im Zusammenhang mit der Beschreibung der erfindungsgemässen Stoffe der Formel (I) vorzugsweise für diese Substituenten genannt wurden. Hal steht vorzugsweise für Fluor, Chlor oder Brom.

Die Halogenide der Formel (III) sind allgemein bekannte Verbindungen der organischen Chemie.

Die ausserdem für das erfindungsgemässe Verfahren (d) als Ausgangsstoffe zu verwendenden Säureanhydride sind durch die Formel (IV) allgemein definiert. In dieser Formel steht $R^5$ vorzugsweise für diejenigen Reste, die bereits im Zusammenhang mit der Beschreibung der erfindungsgemässen Stoffe der Formel (I) vorzugsweise für diesen Substituenten genannt wurden.

Die Säureanhydride der Formel (IV) sind allgemein bekannte Verbindungen der organischen Chemie.

Die ausserdem für das erfindungsgemässe Verfahren (e) als Ausgangsstoffe zu verwendenden Isocyanate sind durch die Formel (V) allgemein definiert. In dieser Formel steht $R^6$ vorzugsweise für diejenigen Reste, die bereits im Zusammenhang mit der Beschreibung der erfindungsgemässen Stoffe der Formel (I) vorzgusweise für diesen Substituenten genannt wurden.

Die Isocyanate der Formel (V) sind allgemein bekannte Verbindungen der organischen Chemie.

Als Lösungsmittel kommen für das erfindungsgemässe Verfahren (a) vorzugsweise polare organische Lösungsmittel infrage. Hierzu gehören vorzugsweise Alkohole, wie Methanol, Ethanol, Isopropanol oder Butanol; und Ether, wie Diethylether oder Tetrahydrofuran.

Die Reaktionstemperaturen können bei der Durchführung des erfindungsgemässen Verfahrens (a) in einem grösseren Bereich variiert werden. Im allgemeinen arbeitet man zwischen 0 bis 60°C, vorzugsweise bei 0 bis 40°C.

Bei der Durchführung des erfindungsgemässen Verfahrens (a) arbeitet man vorzugsweise in äquimolaren Mengen. Die Isolierung der Endprodukte erfolgt in allgemein üblicher Weise, gegebenenfalls auch als Säureadditions-Salz.

Als Lösungsmittel kommen für das erfindungsgemässe Verfahren (b) vorzugsweise inerte organische Lösungsmittel infrage. Hierzu gehören vorzugsweise Amide, wie Dimethylformamid, Sulfoxide, wie Dimethylsulfoxid, sowie Petrolether. Insbesondere kann auch ein Überschuss des jeweils verwendeten anorganischen Säurehalogenids eingesetzt werden.

Als Säurebindemittel kommen für das erfindungsgemässe Verfahren (b) vorzugsweise organische Basen infrage, wie beispielsweise Pyridin und Triethylamin.

Die Reaktionstemperaturen können bei der Durchführung des erfindungsgemässen Verfahrens (b) in einem grösseren Bereich variiert werden. Im allgemeinen arbeitet man zwischen -20 und 100°C, vorzugsweise zwischen -10 und 80°C.

Bei der Durchführung des erfindungsgemässen Verfahrens (b) arbeitet man vorzugsweise in äquimolaren Mengen. Der Einfachheit halber kann das eingesetzte Halogenierungsmittel auch als Lösungsmittel verwendet werden, womit ein entsprechender Überschuss erforderlich wird. Die Isolierung der Verbindungen der Formel (I) erfolgt, indem man überschüssiges Halogenierungsmittel z.B. durch Destillation entfernt, das Reaktionsgemisch mit wässriger Natriumhydrogencarbonatlösung versetzt und das Reaktionsprodukt mit einem organischen Solvens ausschüttelt.

Als Lösungsmittel kommen für das erfindungsgemässe Verfahren (c) vorzugsweise inerte organische Lösungsmittel infrage. Hierzu gehören vorzugsweise Ether, wie Diethylether und Dioxan; aromatische Kohlenwasserstoffe, wie Toluol und Benzol; in einzelnen Fällen auch chlorierte Kohlenwasserstoffe, wie Chloroform, Methylenchlorid oder Tetrachlorkohlenstoff; sowie Ketone, wie Aceton oder Methylethylketon; Nitrile, wie Acetonitril sowie Petrolether. Der Einfachheit halber kann gegebenenfalls auch ein eingesetztes Säurehalogenid als Lösungsmittel verwendet werden, womit ein entsprechender Überschuss erforderlich wird.

Die Reaktionstemperaturen können bei der Durchführung des erfindungsgemässen Verfahrens (c) in einem grösseren Bereich variiert werden. Im allgemeinen arbeitet man zwischen 20 und 150°C, vorzugsweise zwischen 20 und 100°C, bzw. bei der Siedetemperatur des jeweiligen Lösungsmittels.

Das erfindungsgemässe Verfahren (c) kann gegebenenfalls in Gegenwart einer starken Base durchgeführt werden. Hierzu gehören vorzugsweise Alkalimetall-hydride, Alkalimetallamide und Alkalimetall-alkoholate, wie z.B. Natriumhydrid, Natriumamid und Kalium-tert.-butylat.

Das erfindungsgemässe Verfahren (c) kann gegebenenfalls in Gegenwart von Säurebindern (Halogenwasserstoff-Akzeptoren) durchgeführt werden. Hierzu gehören organische Basen, vorzugsweise tertiäre Amine, wie z.B. Triethylamin; ferner anorganische Basen, wie z.B. Alkalihydroxide und Alkalicarbonate.

Bei der Durchführung des erfindungsgemässen Verfahrens (c) setzt man vorzugsweise auf 1 Mol der Verbindungen der Formel (Ia) 1 bis 3 Mol Halogenid der Formel (III) ein. Zur Isolierung der Endprodukte wird z.B. das Reaktionsgemisch vom Lösungsmittel befreit und der Rückstand mit Wasser und einem organischen Lösungsmittel versetzt. Die organische Phase wird abgetrennt und in üblicher Weise aufgearbeitet.

In einer bevorzugten Ausführungsform wird zweckmässigerweise so verfahren, dass man von einer Verbindung der Formel (Ia) ausgeht, letztere in einem geeigneten inerten organischen Lösungsmittel mittels Alkalimetallhydrid oder Alkalimetallamid in das Alkanolat überführt, und letzteres ohne Isolierung sofort mit einem Halogenid der Formel (III) umsetzt, wobei unter Austritt von Alkalihalogenid die erfindungsgemässen Verbindungen der Formel (I) in einem Arbeitsgang erhalten werden.

Als Lösungsmittel kommen für das erfindungsgemässe Verfahren (d) vorzugsweise inerte organische Lösungsmittel infrage. Hierzu gehören vorzugsweise die beim Verfahren (c) aufgezählten Solventien sowie die jeweils verwendeten Säureanhydride der Formel (IV).

Als Katalysatoren können beim Verfahren (d) vorzugsweise alle üblichen sauren und basischen Katalysatoren verwendet werden, wie z.B. Schwefelsäure, Chlorwasserstoff, Bromwasserstoff, Bortrifluorid, Zinkchlorid, Natrimacetat, Natriumbenzoat, Natriumcarbonat, Calciumoxid, Magnesiumoxid, Pyridin und Triethylamin.

Die Reaktionstemperaturen können bei der Durchführung des erfindungsgemässen Verfahrens (d) in einem grösseren Bereich variiert werden. Im allgemeinen arbeitet man zwischen 20 und 150°C, vorzugsweise zwischen 50 und 120°C.

Bei der Durchführung des Verfahrens (d) arbeitet man vorzugsweise mit äquivalenten Mengen an Ausgangsstoffen. Der Einfachheit halber kann man das eingesetzte Säureanhydrid der Formel (IV) auch als Lösungsmittel verwenden, womit ein entsprechender Überschuss erforderlich wird. Die Isolierung der Verbindungen der Formel (I) erfolgt in üblicher Weise.

Als Lösungsmittel kommen für das erfindungsgemässe Verfahren (e) vorzugsweise inerte organische Lösungsmittel infrage. Hierzu gehören vorzugsweise die beim Verfahren (c) aufgezählten Solventen.

Als Katalysatoren können beim Verfahren (e) vorzugsweise verwendet werden tertiäre Basen, wie Triethylamin und Pyridin, oder Zinn-organische Verbindungen, wie Dibutylzinn-dilaurat und Tributylzinn-laurat.

Die Reaktionstemperaturen können bei der Durchführung des erfindungsgemässen Verfahrens (e) in einem grösseren Bereich variiert werden. Im allgemeinen arbeitet man zwischen 0 und 100°C, vorzugsweise bei der Siedetemperatur des verwendeten Lösungsmittels.

Bei der Durchführung des erfindungsgemässen Verfahrens (e) arbeitet man vorzugsweise mit äquivalenten Mengen an Ausgangsstoffen. Zur Isolierung der Verbindungen der Formel (I) wird das Lösungsmittel abdestilliert und der Rückstand nach üblichen Methoden aufgearbeitet.

Die nach den Verfahren (a) bis (e) herstellbaren erfindungsgemässen Stoffe der Formel (I) können in Säureadditions-Salze überführt werden.

Zur Herstellung von physiologisch verträglichen Säureadditionssalzen der Verbindungen der Formel (I) kommen vorzugsweise folgende Säuren in Frage: die Halogenwasserstoffsäuren, wie z.B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäure, wie z.B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z.B. gegen den Erreger des Gersten- bzw. des Getreidemehltaus (Erysiphe graminis); sowie zur Bekämpfung solcher Pilze, die Schorf- und Rostkrankheiten hervorrufen, so zur Bekämpfung von Venturia-Arten, wie z.B. gegen den Erreger des Apfelschorfes (Fusicladium dendriticum) und von Uromyces-Arten, wie z.B. gegen den Erreger des Bohnenrostes (Uromyces phaseoli). Ausserdem zeigen die erfindungsgemässen Wirkstoffe eine gute fungizide in-vitro-Wirkung gegen Fusarium nivale.

In bestimmten Aufwandmengen zeigen die erfindungsgemässen Stoffe auch eine wachstumsregulierende Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind. z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talium, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen,

Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

## Herstellungsbeispiele

### Beispiel 1

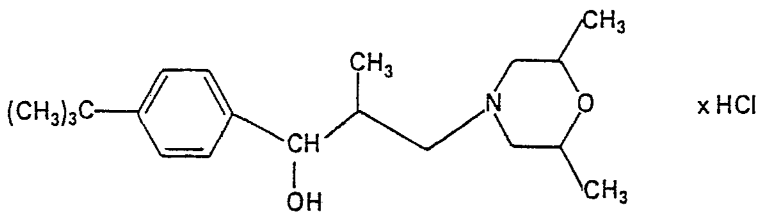

(Verfahren a)

18,3 g (0,06 Mol) p-tert.-Butyl-2-methyl-3-(2,6-dimethylmorpholin-4-yl)propiophenon werden in 150 ml Methanol vorgelegt und portionsweise mit 2,5 g (0,06 Mol) Natriumborhydrid versetzt, wobei die Temperatur 40°C nicht übersteigen soll. Man lässt 3 Stunden bei Raumtemperatur nachrühren, engt ein, nimmt den Rückstand in 100 ml Wasser auf und versetzt mit verdünnter Salzsäure bis zum pH-Wert von 1. Der entstandene Niederschlag wird abgesaugt und getrocknet. Man erhält 14,4 g (67,4% der Theorie) 1-(p-tert.-Butyl-phenyl)-2-methyl-3-(2,6-dimethyl-morpholin-4-yl)-1-propanol-hydrochlorid vom Schmelzpunkt 243°C.

#### Herstellung des Ausgangsproduktes

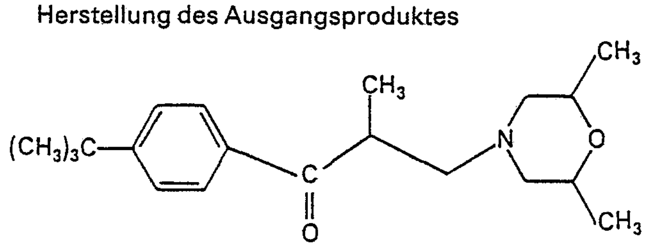

57,6 g (0,5 Mol) 2,6-Dimethylmorpholin-hydrochlorid, 95,2 g (0,5 Mol) p-tert.-Butyl-propiophenon und 25 g (0,83 Mol) Paraformaldehyd werden in 200 ml Ethanol 1 Stunde unter Rückfluss erhitzt. Nach Zugabe von 0,7 ml konzentrierter Salzsäure wird das Reaktionsgemisch weitere 15 Stunden unter Rückfluss gerührt. Danach wird eingeengt, der Rückstand in Chloroform aufgenommen, 2mal mit Wasser gewaschen, über Natriumsulfat getrocknet und eingeengt. Der feste Rückstand wird in heissem Essigester aufgeschlämmt, filtriert und getrocknet. Man erhält 47,6 g (30% der Theorie) p-tert.-Butyl-2-methyl-3-(2,6-dimethylmorpholin-4-yl)-propiphenon-hydrochlorid, das in wässriger Natriumhydrogencarbonatlösung gelöst, anschliessend mit Essigester extrahiert und eingeengt wird. Man erhält quantitativ die freie Base vom Siedepunkt 145-148°C/0,12 mm Hg-Säule.

### Beispiel 2

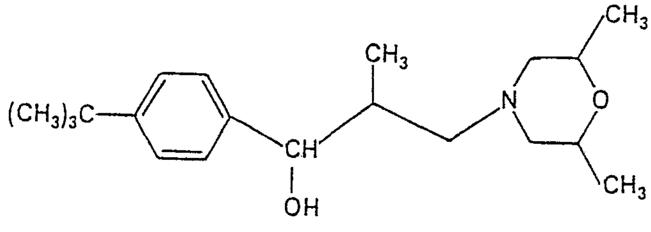

Das nach Beispiel 1 erhaltene Hydrochlorid wird in wässriger Natriumhydrogencarbonatlösung gelöst und anschliessend wird mit Essigester extrahiert und eingeengt. Man erhält quantitativ 1-(p-tert.-Butyl-phenyl)-2-methyl-3-(2,6-dimethyl-morpholin-4-yl)-1-propanol vom Schmelzpunkt 49-50°C.

Beispiel 3

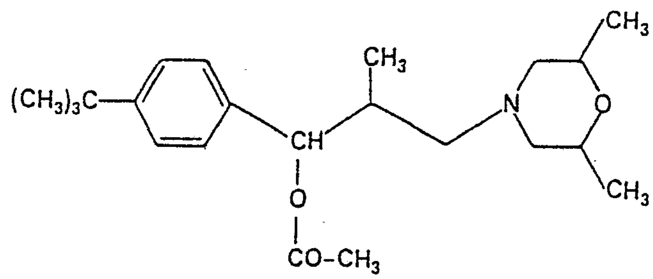

(Verfahren d)

7,2 g (0,02 Mol) 1-(p-tert.-Butyl-phenyl)-2-methyl-3-(2,6-dimethyl-morpholin-4-yl)-1-propanol (Beispiel 2) werden in 50 ml Essigsäureanhydrid 3 Stunden bei Raumtemperatur gerührt. Anschliessend wird das Reaktionsgemisch in 100 ml Wasser gegeben, mit verdünnter Natronlauge auf einen pH-Wert von 7 eingestellt und mit Chloroform extrahiert. Die organische Phase wird über Natriumsulfat getrocknet und eingeengt. Der ölige Rückstand wird in Essigester aufgenommen, mit Aktivkohle geschüttelt, filtriert und das Filtrat eingeengt. Das erhaltene Öl wird mit Petrolether digeriert, von einem braunen Niederschlag abgetrennt und eingeengt. Man erhält 5,3 g (73% der Theorie) 1-Acetoxy-1-(p-tert.-butyl-phenyl)-2-methyl-3-(2,6-dimethyl-morpholin-4-yl)-propan als zähflüssiges Öl.

Beispiel 4

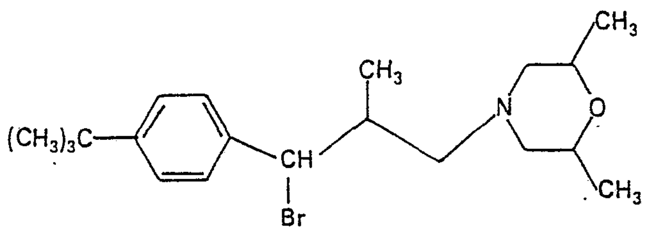

(Verfahren b)

21,6 g (0,07 Mol) 1-(p-tert.-Butyl-phenyl)-2-methyl-3-(2,6-dimethyl-morpholin-4-yl)-1-propanol (Beispiel 2) werden mit 1,9 ml Pyridin in 250 ml Petrolether gelöst und bei -5 bis -10°C wird eine Lösung von 2,9 ml (0,03 Mol) Phosphortribromid in 50 ml Petrolether zugetropft. Es wird 3 Stunden bei Raumtemperatur nachgerührt, der Niederschlag abgesaugt, mit Petrolether gewaschen, und in Chloroform gelöst. Die Chloroformphase wird mit Natriumhydrogencarbonatlösung gewaschen, über Natriumsulfat getrocknet und im Vakuum eingeengt. Man erhält 23 g (88% der Theorie) 1-Brom-1-(p-tert.-Butyl-phenyl)-2-methyl-3-(2,6-dimethylmorpholin-4-yl)-propan als zähflüssiges Öl.

Beispiel 5

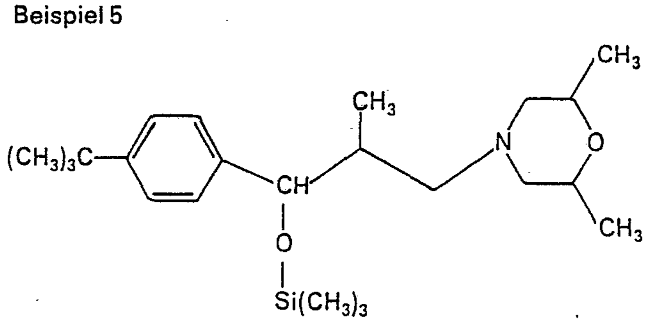

(Verfahren c)

10 g (0,03 Mol) 1-(p-tert.-Butyl-phenyl)-2-methyl-3-(2,6-dimethylmorpholin-4-yl)-1-propanol (Beispiel 2) werden mit 4,4 ml Triethylamin in 160 ml Petrolether bei Raumtemperatur gelöst und tropfenweise mit 3,4 g Trimethylchlorsilan in 40 ml Petrolether versetzt. Der Niederschlag wird abgesaugt und das Filtrat eingeengt. Man erhält 12 g (98% der Theorie) 1-(p-tert.-Butylphenyl)-1-trimethylsilyloxy-2-methyl-3-(2,6-dimethylmorpholin-4-yl)-propan als zähflüssiges Öl.

Beispiel 6

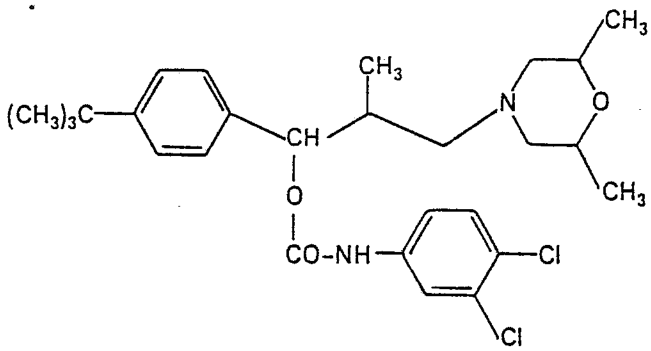

(Verfahren e)
6,8 g (0,021 Mol) 1-(p-tert.-Butyl-phenyl)-2-methyl-3-(2,6-dimethylmorpholin-4-yl)-1-propanol (Beispiel 2) und 0,6 Mol Triethylamin werden in 60 ml Acetonitril gelöst und bei Raumtemperatur tropfenweise mit einer Lösung von 4,0 g 3,4-Dichlorphenylisocyanat in 10 ml Acetonitril versetzt. Man lässt eine Stunde unter Rückfluss nachrühren, kühlt ab und engt ein. Der Rückstand wird in Essigester aufgenommen, mit Wasser gewaschen, über Natriumsulfat getrocknet und eingeengt. Man erhält 8,2 g (70% der Theorie) 1-(p-tert.-Butyl-phenyl)-1-(3,4-dichlorphenyl-carbamoyloxy)-2-methyl-3-(2,6-dimethylmorpholin-4-yl)-propan als zähflüssiges Öl.

In entsprechender Weise und gemäss der angegebenen Verfahren werden die nachfolgenden Beispiele der allgemeinen Formel

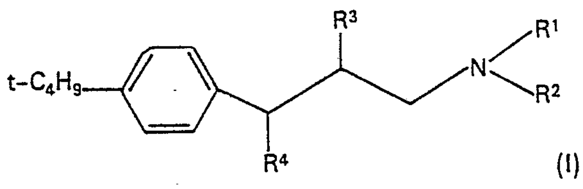

(I)

erhalten:

| Beispiel Nr. | $R^4$ | $R^3$ | 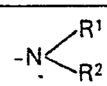 | Schmelzpunkt (°C) |
|---|---|---|---|---|
| 7 | -OH | $CH_3$ | 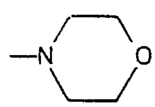 | 253 (Zers.)(x HCl) |
| 8 | -OH | $CH_3$ | 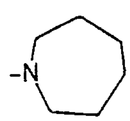 | 228-29 (x HCl) |
| 9 | -OH | $CH_3$ | 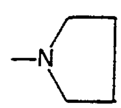 | 211-15 (x HCl) |
| 10 | Cl | $CH_3$ | 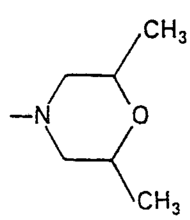 | Öl |
| 11 | 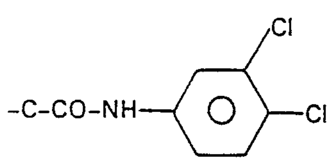 | $CH_3$ | 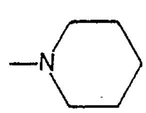 | Öl |

| Beispiel Nr. | $R^4$ | $R^3$ | 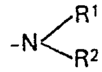 | Schmelzpunkt (°C) |
|---|---|---|---|---|
| 12 | $-O-Si(CH_3)_3$ | $CH_3$ | 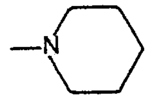 | Öl |
| 13 | OH | $CH_3$ | $-N(C_2H_5)_2$ | 185 (x HCl) |
| 14 | OH | $CH_3$ | $-N(C_3H_7)_2$ | 152 (x HCl) |
| 15 | Cl | $CH_3$ | $-N(C_3H_7)_2$ | 145 |
| 16 | -O-CO-NH- 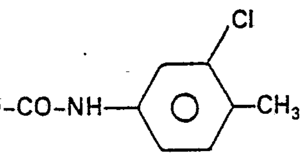 | $CH_3$ | 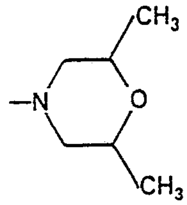 | Öl |
| 17 | -O-CO-NH- 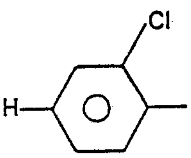 | $CH_3$ | 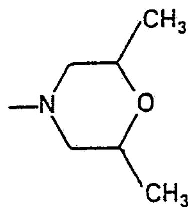 | Öl |
| 18 | -O-CO-NH- 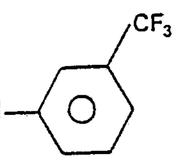 | $CH_3$ | 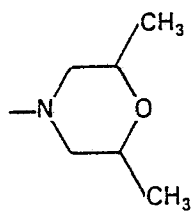 | Öl |
| 19 | $-O-CO-NH-C_3H_7-i$ | $CH_3$ | 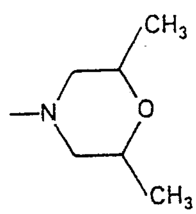 | Öl |
| 20 | $-O-CO-NH-CH_3$ | $CH_3$ | 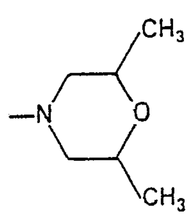 | Öl |
| 21 | $-O-CO-NH-CH_2-C_3H_7-i$ | $CH_3$ | 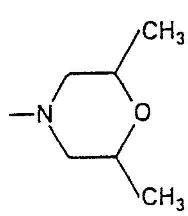 | Öl |

| Beispiel Nr. | $R^4$ | $R^3$ | 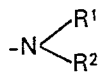 | Schmelzpunkt (°C) |
|---|---|---|---|---|
| 22 | $-O-CO-NH-C_4H_9-n$ | $CH_3$ | 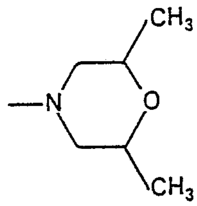 | Öl |
| 23 | $-O-CO-CH_2Cl$ | $CH_3$ | 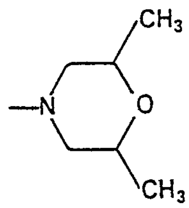 | 174–82 (x HCl) |
| 24 | $-O-CO-CHCl_2$ | $CH_3$ | 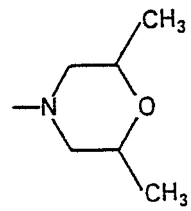 | 171–78 (x HCl) |
| 25 | $-O-Si(CH_3)_3$ | $CH_3$ | 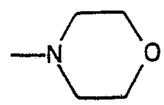 | Öl |
| 26 | CH | $CH_3$ | $-N(C_3H_7-i)_2$ | 206 (x HCl) |
| 27 | CH | $CH_3$ | $-N(-CH_2-C_3H_7-i)_2$ | Öl (x HCl) |
| 28 | CH | $CH_3$ | $-N(C_4H_9-n)_2$ | Öl (x HCl) |

Verwendungsbeispiel

In dem nachfolgenden Beispiel wird die nachstehend angegebene Verbindung als Vergleichssubstanz eingesetzt:

(A) 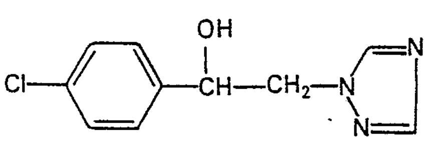

Beispiel A

Erysiphe-Test (Gerste) [protektiv]

Lösungsmittel: 100 Gewichtsteile Dimethylformamid

Emulgator: 0,25 Gewichtsteile Alkyl-aryl-polyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 2, 3, 4 und 7.

**Patentansprüche**

1. Aminopropanol-Derivate der allgemeinen Formel

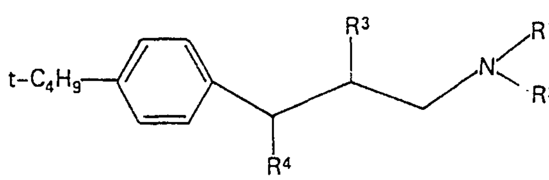

(I)

in welcher
$R^1$ und $R^2$ für Alkyl mit 1 bis 4 Kohlenstoffatomen stehen; oder gemeinsam mit dem Stickstoffatom, an das sie gebunden sind, für die gegebenenfalls durch Alkyl mit 1 bis 4 Kohlenstoffatomen sowie durch einen gegebenenfalls durch Alkyl mit 1 bis 4 Kohlenstoffatomen und Halogen substituierten ankondensierten aromatischen oder alicyclischen Ring mit 5 bis 7 Kohlenstoffatomen substituierten Heterocyclen

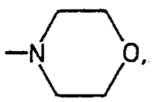 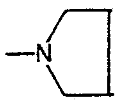 und 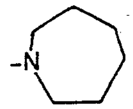

stehen; sowie
zusätzlich auch für gegebenenfalls durch Alkyl mit 1 bis 4 Kohlenstoffatomen sowie durch einen gegebenenfalls durch Alkyl mit 1 bis 4 Kohlenstoffatomen und Halogen substituierten ankondensierten aromatischen oder alicyclischen Ring mit 5 bis 7 Kohlenstoffatomen substituierten Heterocyclus

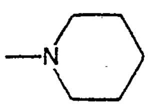

stehen, wenn $R^4$ für die Reste $-O-CO-R^5$, $-O-CO-NHR^6$ oder $-O-SiR_3{}^7$ steht,

$R^3$ für Wasserstoff oder Alkyl mit 1 bis 4 Kohlenstoffatomen steht,

$R^4$ für die Hydroxygruppe, Halogen sowie die Reste $-O-CO-R^5$, $-O-CO-NHR^6$ oder $-O-SiR_3{}^7$ steht,

$R^5$ für Alkyl mit 1 bis 4 Kohlenstoffatomen, Alkenyl mit 2 bis 4 Kohlenstoffatomen, Halogenalkyl mit 1 bis 2 Kohlenstoffatomen und 1 bis 5 gleichen oder verschiedenen Halogenatomen, sowie für gegebenenfalls substituiertes Aryl mit 6 bis 10 Kohlenstoffatomen und Aralkyl mit 6 bis 10 Kohlenstoffatomen im Arylteil und 1 bis 2 Kohlenstoffatomen im Alkylteil, wobei als Substituenten genannt seien: Halogen, Alkyl mit 1 bis 4 Kohlenstoffatomen, Halogenalkyl mit 1 bis 2 Kohlenstoffatomen und 1 bis 5 gleichen oder verschiedenen Halogenatomen, Alkoxy und Alkylthio mit jeweils 1 oder 2 Kohlenstoffatomen steht,

$R^6$ für Alkyl mit 1 bis 8 Kohlenstoffatomen, Alkenyl mit 2 bis 4 Kohlenstoffatomen sowie für gegebenenfalls substituiertes Aryl mit 6 bis 10 Kohlenstoffatomen, wobei als Substituenten die bereits oben genannten Phenylsubstituenten in Frage kommen und

$R^7$ für Alkyl mit 1 bis 4 Kohlenstoffatomen steht und deren physiologisch verträglichen Säureadditionssalze.

2. Verfahren zur Herstellung von Aminopropanol-Derivaten der Formel I in Anspruch 1, dadurch gekennzeichnet, dass man
a) Aminopropiophenone der Formel

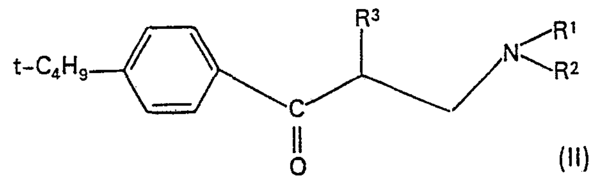

(II)

in welcher
$R^1$, $R^2$ und $R^3$ die in Anspruch 1 angegebene Bedeutung haben,
mit komplexen Hydroiden in Gegenwart eines Lösungsmittels reduziert, und
gegebenenfalls die nach dem Verfahren a) erhältlichen Aminopropanole der Formel

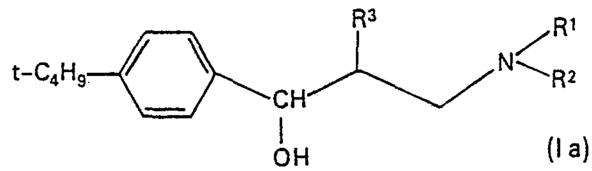

(I a)

b) mit einem Halogenierungsmittel, gegebenenfalls in Gegenwart eines Lösungsmittels, und gegebenenfalls in Gegenwart eines Säurebindemittels, umsetzt, oder
c) mit Halogeniden der Formel

$Hal-R^8$ (III)

in welcher
$R^8$ für die Reste $-CO-R^5$, $-CO-NHR^6$ oder $-SiR_3{}^7$ steht, wobei $R^5$, $R^6$ und $R^7$ die in Anspruch 1 angegebene Bedeutung haben und
Hal für Halogen steht,
in Gegenwart eines Lösungsmittels und gegebenenfalls
in Gegenwart einer starken Base bzw. gegebenenfalls
in Gegenwart eines Säurebindemittels umsetzt, oder
d) mit Säureanhydriden der Formel

$R^5-CO-O-CO-R^5$ (IV)

in welcher
$R^5$ die in Anspruch 1 angegebene Bedeutung hat,
in Gegenwart eines Lösungsmittels und gegebenenfalls
in Gegenwart eines Katalysators umsetzt, oder
e) mit Isocyanaten der Formel

$O=C=N-R^6$ (V)

in welcher
$R^6$ die in Anspruch 1 angegebene Bedeutung hat,
in Gegenwart eines Lösungsmittels und gegebenenfalls in Gegenwart eines Katalysators umsetzt, und gegebenenfalls an die nach obigen Verfahren erhaltenen Verbindungen der Formel (I) eine physiologisch verträgliche Säure addiert.

3. Fungizide Mittel, gekennzeichnet durch einen Gehalt an mindestens einem Aminopropanol-Derivat der Formel I.

4. Verfahren zur Bekämpfung von Pilzen, dadurch gekennzeichnet, dass man Aminopropanol-Derivate der Formel I auf Pilze oder ihren Lebensraum einwirken lässt.

5. Verwendung von Aminopropanol-Derivaten der Formel I zur Bekämpfung von Pilzen.

6. Verfahren zur Herstellung von fungiziden Mitteln, dadurch gekennzeichnet, dass man Aminopropanol-Derivate der Formel I mit Streckmitteln und/oder oberflächenaktiven Mitteln vermischt.

**Claims**

1. Aminopropanol derivatives of the general formula

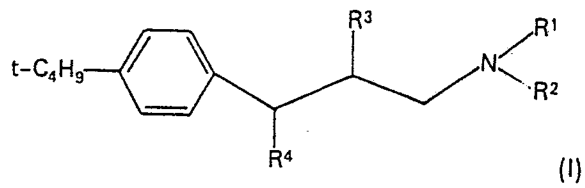

(I)

in which

$R^1$ and $R^2$ represent alkyl with 1 to 4 carbon atoms; or together with the nitrogen atom to which they are bonded represent the heterocyclic radicals

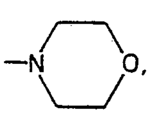, 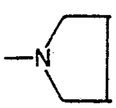 or 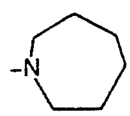

which are optionally substituted by alkyl with 1 to 4 carbon atoms or by a fused-on aromatic or alicyclic ring which has 5 to 7 carbon atoms and is optionally substituted by alkyl with 1 to 4 carbon atoms or halogen; or in addition also represent a heterocyclic radical

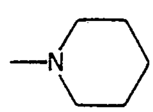

which is optionally substituted by alkyl with 1 to 4 carbon atoms or by a fused-on aromatic or alicyclic ring which has 5 to 7 carbon atoms and is optionally substituted by alkyl with 1 to 4 carbon atoms or halogen, if $R^4$ represents the radicals $-O-CO-R^5$, $O-CO-NHR^6$ or $-O-SiR_3^7$,

$R^3$ represents hydrogen or alkyl with 1 to 4 carbon atoms,

$R^4$ represents the hydroxyl group, halogen or the radicals $-O-CO-R^5$, $-O-CO-NHR^6$ or $-O-SiR_3^7$,

$R^5$ represents alkyl with 1 to 4 carbon atoms, alkenyl with 2 to 4 carbon atoms, halogenoalkyl with 1 to 2 carbon atoms and 1 to 5 identical or different halogen atoms, or optionally substituted aryl with 6 to 10 carbon atoms or aralkyl with 6 to 10 carbon atoms in the aryl part and 1 to 2 carbon atoms in the alkyl part, the substituents which may be mentioned being: halogen, alkyl with 1 to 4 carbon atoms, halogenoalkyl with 1 to 2 carbon atoms and 1 to 5 identical or different halogen atoms, and alkoxy and alkylthio with in each case 1 or 2 carbon atoms,

$R^6$ represents alkyl with 1 to 8 carbon atoms, alkenyl with 2 to 4 carbon atoms or optionally substituted aryl with 6 to 10 carbon atoms, possible substituents being the phenyl substituents already mentioned above and

$R^7$ represents alkyl with 1 to 4 carbon atoms, and physiologically acceptable acid addition salts thereof.

2. Process for the preparation of aminopropanol derivatives of the formula I in Claim 1, characterised in that

a) aminopropiophenones of the formula

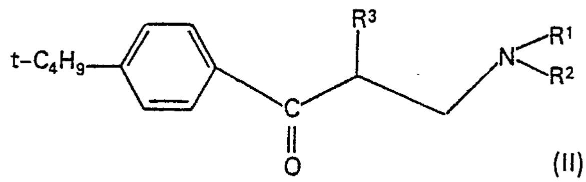

(II)

in which

$R^1$, $R^2$ and $R^3$ have the meaning indicated in Claim 1, are reduced with complex hydrides in the presence of a solvent, and if desired, the aminopropanols of the formula

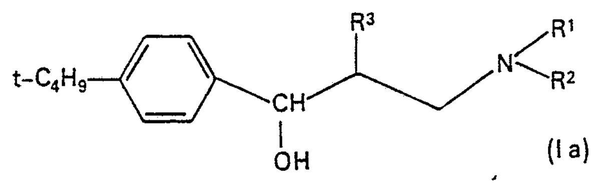

(I a)

obtainable by method a)

b) are reacted with a halogenating agent, if appropriate in the presence of a solvent, and if appropriate in the presence of an acid-binding agent, or

c) are reacted with halides of the formula

$$Hal-R^8 \qquad (III)$$

in which

$R^8$ represents the radicals $-CO-R^5$, $-CO-NHR^6$ or $-SiR_3^7$,

$R^5$, $R^6$ and $R^7$ having the meaning indicated in Claim 1, and Hal represents halogen,

in the presence of a solvent and if appropriate in the presence of a strong base or if appropriate in the presence of an acid-binding agent, or

d) are reacted with acid anhydrides of the formula

$$R^5-CO-O-CO-R^5 \qquad (IV)$$

in which

$R^5$ has the meaning indicated in Claim 1,

in the presence of a solvent and if appropriate in the presence of a catalyst, or

e) are reacted with isocyanates of the formula

$$O=C=N-R^6 \qquad (V)$$

in which
$R^6$ has the meaning indicated in Claim 1, in the presence of a solvent and if appropriate in the presence of a catalyst, and, if desired, a physiologically acceptable acid is added on to the compounds of the formula (I) obtained by the above methods.

3. Fungicidal agents, characterised in that they contain at least one aminopropanol derivative of the formula I.

4. Process for combating fungi, characterised in that aminopropanol derivatives of the formula I are allowed to act on fungi or their habitat.

5. Use of aminopropanol derivatives of the formula I for combating fungi.

6. Process for the preparation of fungidical agents, characterised in that aminopropanol derivatives of the formula I are mixed with extenders and/or surface-active agents.

**Revendications**

1. Derivés de l'aminopropanol de formule générale

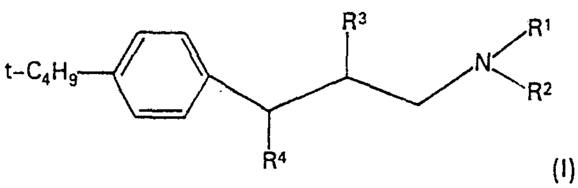

(I)

dans laquelle
$R^1$ et $R^2$ représentent des groupes alkyle en $C_1$–$C_4$; ou forment en commun et avec l'atome d'azote sur lequel ils sont fixés les restes hétérocycliques

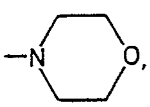, 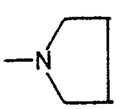 et 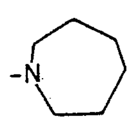

éventuellement substitués par un groupe alkyle en $C_1$–$C_4$ ou par un noyau aromatique ou alicyclique condensé en $C_5$–$C_7$ éventuellement substitué par un groupe alkyle en $C_1$–$C_4$ et par des halogènes; ou encore un reste hétérocyclique

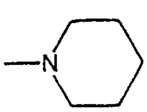

éventuellement substitué par un groupe alkyle en $C_1$–$C_4$ ou par un noyau aromatique ou alicyclique condensé en $C_5$–$C_7$ éventuellement substitué par un groupe alkyle en $C_1$–$C_4$ et par des halogènes lorsque $R^4$ représente les restes -O-CO-$R^5$, -O-CO-NH$R^6$ ou -O-Si$R_3^7$,
$R^3$ représente l'hydrogène ou un groupe alkyle en $C_1$–$C_4$,
$R^4$ représente le groupe hydroxy, un halogène ou les restes -O-CO-$R^5$, -O-CO-NH$R^6$ ou -O-$Si_3^7$,
$R^5$ représente un groupe alkyle en $C_1$–$C_4$, alcényle en $C_2$–$C_4$, halogénoalkyle contenant 1 à 2 atomes de carbone et 1 à 5 atomes d'halogènes identiques ou différents, ou un groupe aryle éventuellement substitué en $C_6$–$C_{10}$ et aralkyle contenant 6 à 10 atomes de carbone dans la partie aryle et 1 à 2 atomes de carbone dans la partie alkyle, les substituants pouvant consister en halogènes, groupes alkyle en $C_1$–$C_4$, halogénoalkyle contenant 1 à 2 atomes de carbone et 1 à 5 atomes d'halogènes identiques ou différents, alcoxy et alkylthio contenant chacun 1 ou 2 atomes de carbone,
$R^6$ représente un groupe alkyle en $C_1$–$C_8$, alcényle en $C_2$–$C_4$ ou aryle en $C_6$–$C_{10}$ éventuellement substitué, les substituants étant ceux déjà cités ci-dessus en tant que substituants du groupe phényle, et
$R^7$ représente un groupe alkyle en $C_1$–$C_4$,
et leurs sels formés par addition avec des acides tolérés par l'organisme.

2. Procédé de préparation de dérivés de l'aminopropanol de formule I de la revendication 1, caractérisé en ce que:
a) on réduit des aminopropiophénones de formule

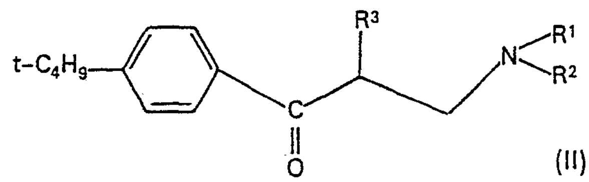

(II)

dans laquelle
$R^1$, $R^2$ et $R^3$ ont les significations indiquées dans la revendications 1, à l'aide d'hydrures complexes en présence d'un solvant, et, le cas échéant, on fait réagir les aminopropanols de formule

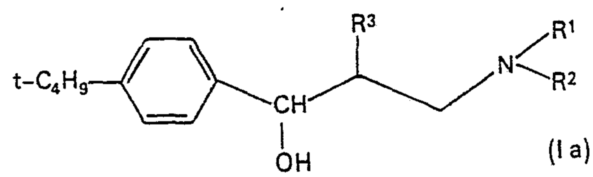

(I a)

obtenus par le procédé a),
b) avec un agent halogénant, éventuellement en présence d'un solvant et éventuellement en présence d'un agent fixant les acides, ou bien
c) avec des halogénures de formule

Hal-$R^8$ (III)

dans laquelle
$R^8$ représente les restes -CO-$R^5$, -CO-NH$R^6$ ou Si$R_3^7$, $R^5$, $R^6$ et $R^7$ ayant les significations indiquées dans la revendication 1, et Hal représente un halogène,
en présence d'un solvant et éventuellement en présence d'une base forte ou éventuellement en présence d'un agent fixant les acides, ou bien
d) avec des anhydrides de formule

$R^5$-CO-O-CO-$R^5$ (IV)

dans laquelle
$R^5$ a la signification indiquée dans la revendication 1,
en présence d'un solvant et, le cas échéant, en présence d'un catalyseur, ou bien

e) avec des isocyanates de formule

$$O=C=N-R^6 \quad (V)$$

dans laquelle
R6 a la signification indiquée dans la revendication 1,
en présence d'un solvant et, le cas échéant, en présence d'un catalyseur et, le cas échéant, sur les composés de formule I obtenus par les procédés ci-dessus, on fixe par addition un acide toléré par l'organisme.

3. Produit fongicide, caractérisé en ce qu'il contient au moins un dérivé de l'aminopropanol de formule I.

4. Procédé pour combattre les champignons, caractérisé en ce que l'on fait agir des dérivés de l'aminopropanol de formule I sur les mycètes ou leur habitat.

5. Utilisation des dérivés de l'aminopropanol de formule I dans la lutte contre les champignons.

6. Procédé de préparation de produits fongicides, caractérisé en ce que l'on mélange des dérivés de l'aminopropanol de formule I avec des diluants et/ou des agents tensio-actifs.